# EUROPEAN PATENT APPLICATION

(11) **EP 4 194 034 A1**
(43) Date of publication of application: **14.06.2023**
(21) Application number: 21306757.2
(22) Date of filing: 13.12.2021
(51) Int. Cl.: A61M 5/31, A61M 5/32

(54) **NEEDLE COVER HAVING ROUGH SURFACE**

(71) Applicant: Becton Dickinson France, 38800 Le Pont-de-Claix (FR)
(72) Inventor: TACHEGOUSTE, Nora, 38000 Grenoble (FR); LAVIGNE, Ferdinand, 38170 Seyssinet Pariset (FR); LEHEE, Guillaume, 38340 Voreppe (FR); POUGET, Gaelle, 38100 Grenoble (FR)
(74) Representative: Regimbeau

(57) **Abstract**

A needle cover for an injection device comprises a needle cover body having a cavity therein. The cavity configured to receive a distal end of an injection device therein and having at least a portion configured such that, when the needle cover is disposed on an injection device, the portion at least partially engages with a distal end of the injection device. The inner surface defining the portions has a surface roughness extending in a range from 2 µm to 12 µm, inclusive. An injective device having such a needle cover and a method of forming the needle cover are also disclosed.

## Description

### TECHNICAL FIELD

The present disclosure relates generally to a needle cover to be disposed on a distal end of an injection device prior to use of the injection device. The needle cover comprises a rough surface configured to reduce the pull out force of the needle cover relative to the injection device.

### BACKGROUND

Injection devices, such as syringes, are medical delivery devices configured to administer a medicinal fluid (e.g., pharmaceutical, medicament) to a patient. Syringes may be sold in a prefilled form, wherein medicinal fluid is disposed within the syringe at, for example, the time of manufacture of the syringe. Syringes typically include a container for containing the medicinal fluid having an end piece including a fluid passageway through which the medicinal fluid is expelled from the container.

In some instances, a needle may be attached to the end piece of the syringe in order to prick a patient's skin and administer the medicinal fluid to the patient. A needle cover may be mounted on the end piece so as to enclose the needle. The needle cover may comprise an inner needle shield, which may be made from an elastomeric material, and may further comprise an outer needle shield, which may be formed of a rigid material. The inner needle shield is disposed in (e.g., inserted in) the outer needle shield.

The needle cover may prevent injury to a person handling the injection device. Further, the inner needle shield may sealingly contact the needle and/or the end piece in order to shield the needle and the end piece from any contamination of the medicinal fluid from an outside environment and/or in order to prevent leakage of the medicinal fluid from the syringe.

In order to use the syringe, the needle cover must be removed. In some instances, the needle cover may be directly gripped by the user. Alternatively, the syringe may be used in combination with an autoinjector or other device in which the needle cover is not directly accessible by the user. In such cases, the needle cover may be operatively coupled to a protective cap, which will be gripped by the user to remove the protective cap and the needle cover coupled thereto. The user must apply a sufficient pulling force to separate the needle cover from the syringe. Some users may consider this pulling force to be significant.

The force needed to remove the needle cover from the injection device (e.g., syringe) is measured by a physical parameter known as "pull out force," which may be abbreviated as "POF." In order for the injection device to be usable by any user, including users having a reduced strength due to, for example, a disease, the pull out force must be sufficiently low. However, the pull out force must also be sufficiently high so as to avoid unintentional removal of the needle cover.

### BRIEF SUMMARY

An objective of the present disclosure is to provide a needle cover that has a reduced pull out force when separated from a syringe as compared to a conventional needle cover.

Further, the needle cover sealingly engages the syringe on which the needle cover is disposed. Such engagement prevents any contamination of the medicinal fluid disposed within the syringe from the outside environment, thereby assuring the container closure integrity. The needle cover further prevents any leakage of the medicinal fluid from the syringe. To that end, the needle cover having a reduced pull out force must maintain the container closure integrity over time, even after a long storage period.

To this end, the invention proposes a needle cover for an injection device comprising a needle cover body having a longitudinal axis extending between a proximal end and a distal end. The needle cover body has a cavity extending into the needle cover body from the proximal end thereof. The cavity is defined by an inner surface of the needle cover body and is configured to receive a distal end of an injection device therein. The cavity comprises a first portion having a width measured perpendicular to the longitudinal axis and a second portion located distally relative to the first portion and having a width measured perpendicular to the longitudinal axis that is less than the width of the first portion. The first portion and the second portion are configured such that, when the needle cover is disposed on an injection device, the second portion is at least partially engaged with a distal end of the injection device. The inner surface of the needle cover body defining the second portion has a surface roughness extending in a range from 2 µm to 12 µm, inclusive.

Certain preferred but non-limiting features of the needle cover described above are the following, taken individually or in combination:
the inner surface defining the second portion comprises a plurality of reliefs extending into the needle cover body;
the plurality of reliefs are distributed along a length of the second portion and about a perimeter of the second portion;
the plurality of reliefs are randomly distributed along the inner surface of the needle cover body;
the plurality of reliefs are orderly distributed along the inner surface of the needle cover body;
the needle cover body comprises an elastomeric material;
the cavity further comprises a third portion located distally relative to the second portion, the third portion configured such that, when the needle cover is disposed on the injection device, the third portion is sealingly engaged with a needle of the injection device;
the inner surface of the needle cover body of the first portion is a smooth surface, the smooth surface having a surface roughness greater than or equal to 0 µm and less than 2 µm;
the inner surface of the needle cover body of the first portion comprises a plurality of features different from the plurality of reliefs such that the inner surface of the first portion has a surface roughness extending in a range from 2 µm to 100 µm, inclusive; and/or
the needle cover further comprises a rigid shield in which the needle cover body is disposed.

The invention also proposes an injection device comprising a syringe comprising a barrel having a medicament fluid disposed therein, a hub disposed at a distal end of the barrel, and a needle coupled to a distal end of the hub; and the needle cover comprising features of the needle cover described above, taken individually or in combination, that is disposed on a distal end of the syringe such that at least a part of the inner surface of the needle cover body is in contact with and sealingly engaged with the needle and an exterior surface of the distal end of the syringe.

Certain preferred but non-limiting features of the injection device described above are the following, taken individually or in combination:
the syringe comprises glass; and/or
a pull out force for removing the needle cover from the syringe is in a range from 7 N to 16 N, preferably 7-10N or 10-16 N, when measured between 0 and 90 days after the needle cover is subjected to a sterilization process.

The invention also proposes a method of forming the needle cover comprising features of the needle cover described above, taken individually or in combination, comprising injection molding the needle cover body using a mold having a surface roughness in a range from 2 µm to 12 µm, inclusive.

### BRIEF DESCRIPTION OF THE DRAWINGS

The illustrations presented herein are not meant to be actual views of any particular component, device, or system, but are merely idealized representations which are employed to describe embodiments of the present invention. Other features, goals, and advantages of the present invention will appear more clearly upon reading of the detailed description that follows and with references to drawings provided by way of non-limiting examples wherein:
FIG. 1 is a cross-sectional view of a needle cover as described herein and a portion of a medical device on which the needle cover may be disposed;
FIGS. 2 and 3 are schematic, cross-sectional views of a needle cover having a rough surface as described herein;
FIGS. 4a and 4b are optical images of cross-sections of the needle cover;
FIGS. 5 and 6 are a side view and cross-sectional view, respectively, of an injection device having the needle cover of any of FIGS. 1-3 thereon; and
FIG. 7 is a plot of the surface roughness of the rough surface formed on the needle cover;
FIG. 8 is a plot of the pull out force for needle covers having rough surfaces thereon as measured 0 days after a sterilization process; and
FIG. 9 is a plot of the pull out force for needle covers having rough surfaces thereon as measured 90 days after a sterilization process.

### DETAILED DESCRIPTION

As used herein, the term "proximal" refers to a location, such as a proximal end, that is nearer to a point of reference such as a point of contact of a user applying a force to a plunger rod of an injection device as described herein. As used herein, the term "distal" refers to a location, such as a distal end, that is farther from a point of reference such as a point of contact of the user applying a force to the plunger of the injection device as described herein. Thus, the terms "proximal" and "distal" refer to, for example, directions nearer to and farther from, respectively a user administering a medicinal fluid to a patient.

As used herein, the terms "axial," "axially," "longitudinal," and "longitudinally" generally mean and refer to a direction along or parallel to a longitudinal axis of an element(s) of the injection device described herein.

As used herein, the terms "radial," "radially," "lateral," and "laterally" generally mean and refer to a direction perpendicular to the central, longitudinal axis of the element(s) of the injection device described herein.

As used herein, the term "and/or" includes any and all combinations of one or more of the associated listed items.

As used herein, the term "configured" refers to a size, shape, material composition, material distribution, orientation, and arrangement of one or more of at least one structure and at least one apparatus facilitating operation of one or more of the structure and the apparatus in a pre-determined way.

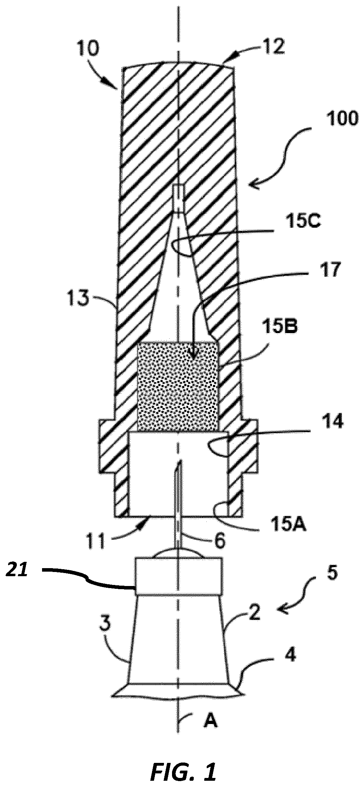

FIGS. 1-3 schematically illustrate needle covers for an injection device. FIG. 1, in particular, illustrates a portion of an injection device 100 on which the needle cover may be disposed.

A needle cover 10 may comprise a needle cover body 12. The needle cover body 12 has a longitudinal axis A extending between a proximal end 20 and a distal end 22 thereof.

The needle cover body 12 has a cavity 11 extending into the needle cover body 12 from the proximal end 20 thereof. The cavity 11 is defined by an inner surface 14 of the needle cover body 12.

The cavity 11 may be configured to receive at least partially a distal end of an injection device 100 therein. To this end, the cavity 11 may be shaped to receive and at least partially engage (e.g., contact) features located at the distal end of the injection device such as the needle 6 and/or a bulge 21.

The cavity 11 may comprise a first portion 15A and a second portion 15B. The second portion 15B may be located distally relative to the first portion 15A. The first portion 15A and the second portion 15B may be configured such that, when the needle cover 10 is disposed on an injection device, the second portion 15B may be at least partially engaged with a distal end of the injection device.

As explained in further detail below, the second portion 15B may be engaged with a hub 2 of the injection device 100, and more preferably with the bulge 21 of the injection device 100.

Preferably, the first portion 15A has a substantially cylindrical shape. Preferably, the second portion 15B has a substantially cylindrical shape. More preferably, both first and second portions 15A and 15B have a substantially cylindrical shape.

A width (e.g., a lateral dimension) of each of the first and second portions 15A, 15B may be measured perpendicular to the longitudinal axis A. The width of the second portion 15B may be less than the width of the first portion 15A. When the first and second portions 15A and 15B have cylindrical shapes, the width may be defined as the diameter of the first and second portions 15A and 15B.

The cavity 11 may, optionally, further comprise a third portion 15C. The third portion 15C may be located distally relative to the first and second portions 15A, 15B. The third portion 15C may be configured such that, when the needle cover 10 is disposed on the injection device 100, the third portion 15C is at least partially engaged with the needle 6 of the injection device 100. More particularly, the needle cover body 12 may be pierced by and, thereby, sealingly engage the needle 6 when the needle cover 10 is disposed on the injection device. The third portion 15C may preferably have a conical shape.

A portion of the inner surface 14 of the needle cover body 12 may exhibit a surface roughness that contributes to a reduced pull out force relative to a needle cover with a smooth surface. As used herein, the term "smooth surface" means a surface of the needle cover having a surface roughness greater than or equal to 0 µm and less than 2 µm.

In some embodiments, the inner surface 14 of the needle cover body 12 defining the second portion 15B may have a surface roughness extending in a range from 2 µm to 12 µm, inclusive.

The foregoing surface roughnesses may be measured using an optical roughometer. More particularly, the measurements may be completed with the following equipment:
□ Equipment: Form Talysurf Inductive measuring system with Ultra Analysis software
□ Resolution: 16 nm
□ Diamond stylus: 90° x 2 µm tip
□ Force: adjustable from 0.7 mN to 1 mN and more, particularly, using 0.75 mN

To create this surface roughness, the inner surface 14 defining the second portion 15B may comprise a plurality of reliefs 17.

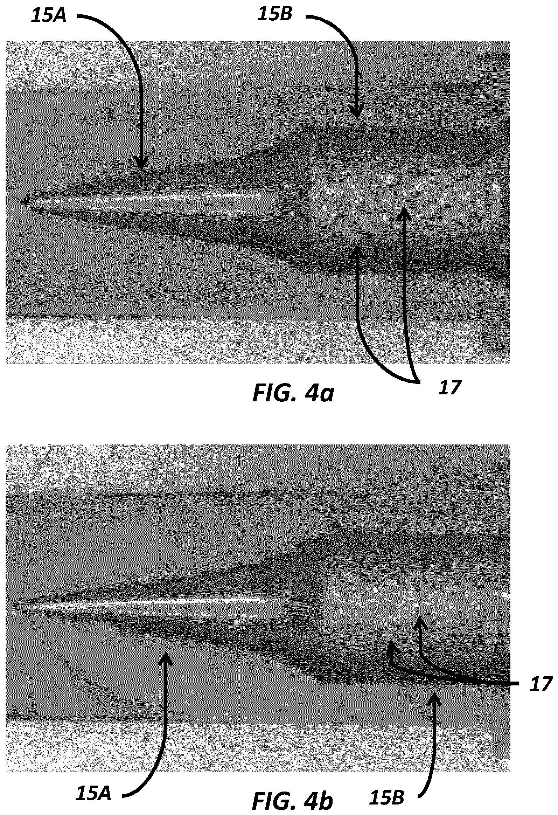

FIGS. 4a and 4b provide a more detailed view of the plurality of reliefs 17 on the inner surface 14. The size and distribution of the plurality of reliefs 17 varies between the needle cover body 12a illustrated in FIG. 4a and the needle cover body 12b illustrated in FIG. 4b.

The reliefs 17 may be concavities extending into the inner surface 14 of the needle cover body 12 and away from the longitudinal axis A of the needle cover body 12. Such reliefs 17 reduce the surface area of the inner surface 14 that engages with (e.g., contacts) an exterior surface of the distal end of the injection device.

The plurality of reliefs 17 may be distributed axially along and circumferentially about the inner surface 14 within the second portion 15B.

In some embodiments, the plurality of reliefs 17 may be randomly distributed along the inner surface 14 of the needle cover body 12. In other embodiments, the plurality of reliefs 17 may be orderly distributed along the inner surface 14 of the needle cover body 12.

The plurality of reliefs 17 may be similar in shape. In some embodiments, the portion of the inner surface 14 defining respective reliefs 17 may be generally hemispherical in shape. In other embodiments, the portion of the inner surface 14 defining respective reliefs 17 may have an irregular shape.

The plurality of reliefs 17 may vary in size such that some reliefs have a height (e.g., depth) and/or a width (e.g., diameter) different from a height and/or width of other reliefs.

Some portion or all of the reliefs 17 may be contiguous and/or some portion or all of the reliefs 17 may be distinct.

The needle cover body 12 may be formed of an elastomeric material. For example, the needle cover body 12 may be formed of rubber or a thermoplastic elastomer.

The needle cover body 12 may be formed by molding, and the reliefs 17 may be formed in the inner surface 14 during the molding process. More particularly, the needle cover body 12 may be formed by an injection molding process.

To form the needle cover body 12, the mold may comprise a plurality of projections that form the reliefs. The size and/or distribution of the projections may be varied in order to modify the surface roughness of the inner surface 14.

In some embodiments, as illustrated in FIG. 1, the first portion 15A and the third portion 15C on the inner surface 14 may have a smooth surface, while the second portion 15B comprises the plurality of reliefs 17.

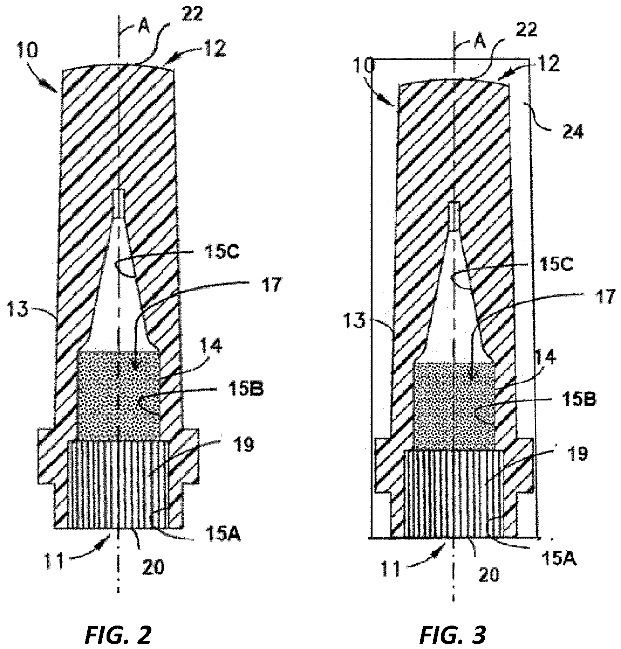

In other embodiments, as illustrated in FIG. 2, the first portion 15A on the inner surface 14 may comprise a plurality of features 19. The features 19 reduce the surface area of the inner surface 14 that engages with an exterior surface of the distal end of the injection device.

The features 19 may be different from the reliefs 17. For example, the features 19 may have a different size and/or shape from the reliefs 17.

In some embodiments, the features 19 may comprise grooves extending axially along inner surface 14 and parallel to the longitudinal axis A. In other embodiments, the features 19 may comprise grooves extending axially and helically along the inner surface 14 such that the groove are inclined (e.g., not parallel) to the longitudinal axis A.

The features 19 may be concavities extending into the inner surface 14 of the needle cover body 12 and away from the longitudinal axis A of the needle cover body 12. Like the reliefs 17, the features 19 reduce the surface area of the inner surface 14 that engages with an exterior surface of the distal end of the injection device.

The inner surface 14 of the needle cover body 12 defining the first portion 15A may have a surface roughness extending in a range from 2 µm to 100 µm, inclusive.

In either of the foregoing embodiments, the inner surface 14 of the needle cover body 12 of the third portion 15C may be a smooth surface.

As illustrated in FIG. 3, the needle cover 10 may further comprise a rigid needle shield 24 in which the needle cover body 12 as described with reference to either FIG. 1 or FIG. 2 may be disposed. The rigid shield 24 may be made of a polymeric material that is more rigid (e.g., having a higher modulus of elasticity) compared to the polymeric material of the needle cover body 12. For instance, the rigid shield 24 may be made of polypropylene.

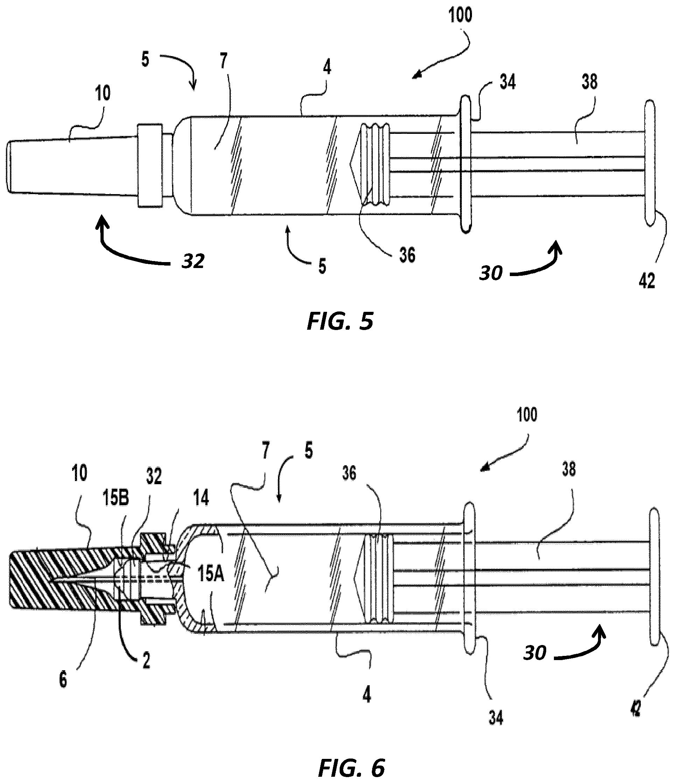

FIGS. 5 and 6 illustrate a side view and a cross-sectional view, respectively, of an injection device 100. The injection device 100 may comprise the needle cover 10 as previously described herein.

The injection device 100 may comprise a syringe 5. The syringe 5 may comprise a barrel 4 defining a cavity 7 in which a medicinal fluid may be disposed. The barrel 4 may extend between a proximal end 30 and a distal end 32. The syringe 5 may have a flanged portion 34 at the proximal end 30. A hub 2 may be disposed at a distal end 32 of the syringe 5. A needle 6 may be coupled to the hub 2.

The hub 2 at the distal end 32 may comprise the bulge 21. The bulge 21 refers to the portion of the hub 2 having the largest width (e.g., a lateral dimension) relative to a remainder of the hub 2.

A stopper 36 and a plunger rod 38 may be disposed in the cavity 7 of the barrel 4. The plunger rod 38 may comprise a proximal end 42 to which a user may apply a force to axially displace the plunger rod and stopper 36 within the barrel 4.

The needle cover 10 may be disposed on the distal end 32 of the syringe 5 such that the inner surface 14 of the needle cover body 12 is at least partially in contact with and sealingly engaged with the needle 6 and an exterior surface 3 of the hub 2. In some embodiments, only the second portion 15B partially engages with the distal hub 2 of the syringe 5, and more precisely with the bulge 21 of the syringe 5.

The barrel 4 may be formed of glass.

In operation, the needle cover 10 may be removed from the distal end 32 of the syringe 5 before a medicinal fluid within the barrel 4 may be administered to a patient. According to the present disclosure, the pull out force (POF) for removing the needle cover 10 from the syringe 5 may be in a range from 7 N to 16 N, inclusive, and preferably may be in a range from 7 N to 10 N or from 10 N to 16 N, inclusive. The foregoing POF are measured between 0 and 90 days after the needle cover 10 and syringe 5 are subjected to an ethylene oxide (EtO) sterilization process.

Such a sterilization process may be applied in the normal course of preparing injection devices for use within the pharmaceutical industry.

### EXPERIMENTAL DATA

The following experimental data illustrates, by way of comparison, the effect of varying a surface roughness of a portion of an inner surface of a needle cover according to the present disclosure on pull out force of the needle cover from a syringe. The portion of the inner surface of the needle cover body having the tested surface roughness was a portion that extends circumferentially about a hub located at a distal end of a syringe and to which a needle is attached. This portion corresponds to the portion 15B of the needle cover described above.

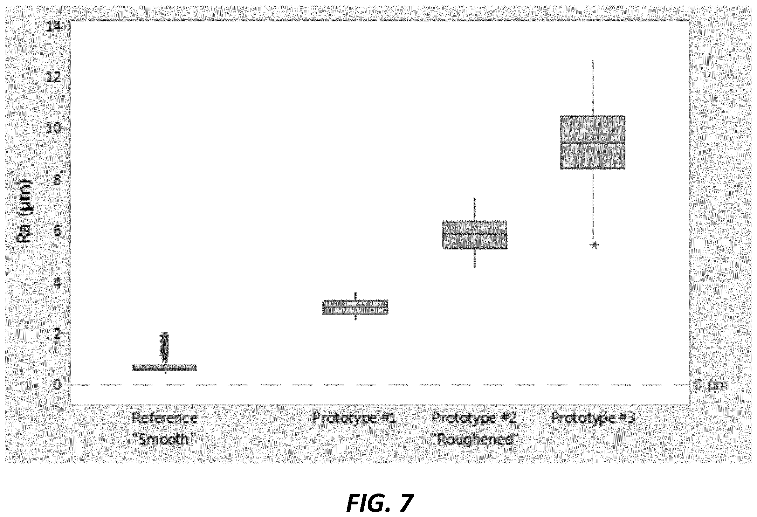

FIG. 7 demonstrates a distribution of surface roughness values for samples of each of the Prototypes. As shown in FIG. 7, experimental data is provided for the following samples:
- Reference: needle covers having a smooth surface (e.g., surface roughness greater than 0 µm but less than 1 µm)
- Prototype #1: needle covers having a surface roughness of about 3 µm
- Prototype #2: needle covers having a surface roughness of about 6 µm
- Prototype #3: needle covers having a surface roughness of about 10 µm

Thirty (30) samples of each of Prototype #1, Prototype #2, and Prototype #3 were tested. For each of the foregoing sample families, the needle cover was made of rubber, the tested surface roughness was formed during a molding process, and the needle cover was disposed on a distal end of a glass syringe and subjected to an ethylene oxide sterilization process.

### Pull off force

Measurements of the force needed to remove the needle cover from a glass syringe were carried out. The pull off force test was performed with a traction bench. The method comprises the steps of:
- placing the syringe on a holder,
- holding the needle cover with pneumatic jaws, and
- subsequently, pulling the needle cover at a constant displacement rate to remove it.

The force needed to remove the needle cover body is recorded, as a function of the displacement of the needle cover. Measurements of the force need to remove the needle cover from the syringe were carried out according to ISO standard 11040-4:2015 Annex G.6 with two exceptions: 1) the needle cover was oriented in a downward direction rather than an upward direction as set forth in the aforementioned ISO standard and 2) the grip length was greater than 2 mm while the ISO standard utilizes a 2 mm grip length.

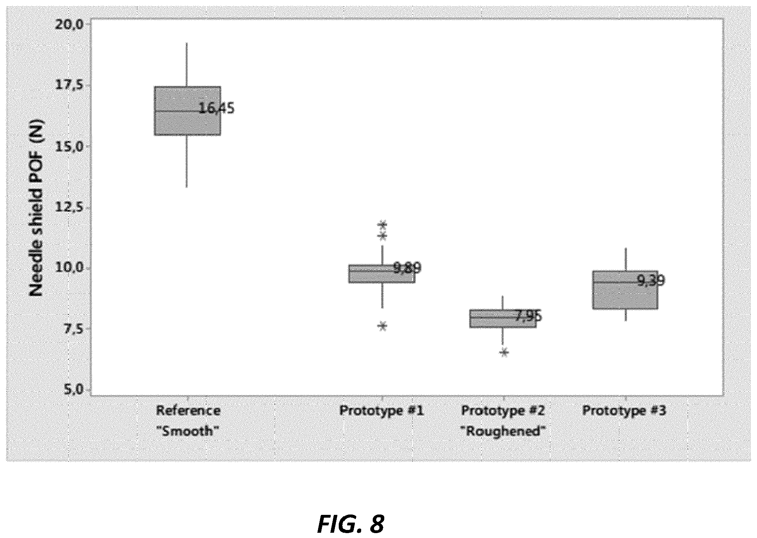

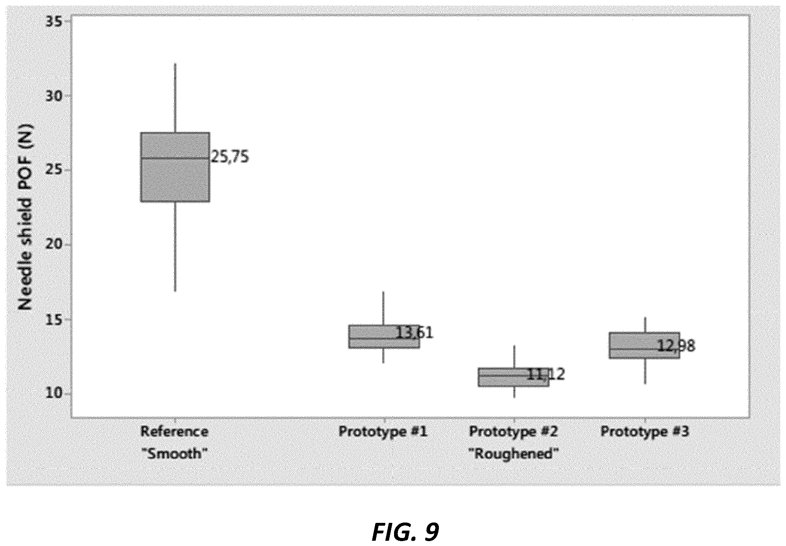

The foregoing samples were tested at two times: a) on the same day as (i.e., 0 days) and following the EtO sterilization (FIG. 8) and b) three months (i.e., 90 days) after the EtO sterilization (FIG. 9). As can be seen from the table below and from FIG. 8, the POF can be reduced by between approximately 40 to 50% for needle covers having rough surfaces compared to needle covers having smooth surfaces, which reduced POF is maintained after ageing.

| SAMPLE | PULL OUT FORCE (N) 0 DAYS AFTER STERLIZATION | PULL OUT FORCE (N) 90 DAYS AFTER STERLIZATION |
|---|---|---|
| Reference | 16.5 | 25.8 |
| Prototype 1 | 9.9 | 13.6 |
| Prototype 2 | 8.0 | 11.1 |
| Prototype 3 | 9.4 | 13.0 |

### Liquid leakage resistance test

Each of Prototype #1-#3 were tested for liquid leakage resistance based upon ISO Standard 11040-42015(E) §G.2. In this test, syringes were filled with sterile water for injection (WFI) and a pressure 1.1 bar for a duration of 5 seconds is applied to the system when the needle cover according to Prototype #1, Prototype #2, and Prototype #3 is disposed on a distal end of a glass syringe. Samples were inspected for leakage occurrence at the interface between the distal end of the syringe and the needle cover.

30 leak assays were carried out on each of Prototype #1, Prototype #2, and Prototype #3 to assess the sealing performance of the needle cover provided with a rough surface.

The results of the test indicate no leaks were found for any assays of Prototype #1, Prototype #2, or Prototype #3.

### Container closure integrity test

Each of Prototype #1-#3 were also subject to container closure integrity testing using a vacuum decay leak detection method based upon ISO Standard 11040-4:2015. In this test, syringes having the needle covers according to Prototype #1, Prototype #2, and Prototype #3 disposed on a distal end of a glass syringe are subject to a vacuum. The test employed a dye solution tightness test which is a destructive approach for detecting leaks in a system. The test is completed in a dye solution. A vacuum pressure is applied on the syringe to verify that no leakage (e.g., product contamination from the environment) occurs at an interface between the needle cover and the glass, stacked needle barrel.

Samples were inspected for leakage occurrence at the interface between the distal end of the syringe and the needle cover.

60 assays were carried out on each of Prototype #1, Prototype #2, and Prototype #3 to assess the leakage resistance of the environment into the needle cover provided with a rough surface.

The results of the test indicated no leaks were found for any assays of Prototype #1, Prototype #2, or Prototype #3.

As previously discussed, in order to ensure the container closure integrity, no leaks of liquid into or out of the syringe should be observed. In view of the foregoing test, the rough surface does not impact the container closure integrity.

While the present disclosure has been described herein with respect to certain illustrated embodiments, those of ordinary skill in the art will recognize and appreciate that it is not so limited. Rather, many additions, deletions, and modifications to the illustrated embodiments may be made without departing from the scope of the disclosure, including legal equivalents thereof. In addition, features from one embodiment may be combined with features of another embodiment while still being encompassed within the scope of the invention as contemplated by the inventors.

## Claims

1. A needle cover (10) for an injection device, comprising:
a needle cover body (12) having a longitudinal axis (A) extending between a proximal end (20) and a distal end (22), the needle cover body (12) having a cavity (11) extending into the needle cover body (12) from the proximal end (20) thereof, the cavity (11) defined by an inner surface (14) of the needle cover body (12), the cavity (11) configured to receive a distal end of an injection device therein, the cavity (11) comprising:
a first portion (15A) having a width measured perpendicular to the longitudinal axis (A); and
a second portion (15B) located distally relative to the first portion (15A) and having a width measured perpendicular to the longitudinal axis (A) that is less than the width of the first portion (15A), the first portion (15A) and the second portion (15B) configured such that, when the needle cover is disposed on an injection device, the second portion (15B) are at least partially engaged with a distal end of the injection device;
wherein the inner surface (14) of the needle cover body (12) defining the second portion (15B) has a surface roughness extending in a range from 2 µm to 12 µm, inclusive.

2. The needle cover (10) of claim 1, wherein the inner surface (14) defining the second portion (15B) comprises a plurality of reliefs (17) extending into the needle cover body (12).

3. The needle cover (10) of claim 2, wherein the plurality of reliefs (17) are distributed along a length of the second portion (15B) and about a perimeter of the second portion (15B).

4. The needle cover (10) of either of claims 2 or 3, wherein the plurality of reliefs (17) are randomly distributed along the inner surface (14) of the needle cover body (12).

5. The needle cover (10) of either of claims 2 or 3, wherein the plurality of reliefs (17) are orderly distributed along the inner surface (14) of the needle cover body (12).

6. The needle cover (10) of any of claims 1-5, wherein the needle cover body (12) comprises an elastomeric material.

7. The needle cover (10) of any of claims 1-6, wherein the cavity (11) further comprises a third portion (15C) located distally relative to the second portion (15B), the third portion (15C) configured such that, when the needle cover is disposed on the injection device, the third portion (15C) is sealingly engaged with a needle of the injection device.

8. The needle cover (10) of any of claims 1-7, wherein the inner surface (14) of the needle cover body (12) of the first portion (15A) is a smooth surface, the smooth surface having a surface roughness greater than or equal to 0 µm and less than 2 µm.

9. The needle cover (10) of any of claims 1-7, wherein the inner surface (14) of the needle cover body (12) of the first portion (15A) comprises a plurality of features (19) different from the plurality of reliefs (17) such that the inner surface (14) of the first portion (15A) has a surface roughness extending in a range from 2 µm to 100 µm, inclusive.

10. The needle cover (10) of any of claims 1-9, further comprising a rigid shield (24) in which the needle cover body (12) is disposed.

11. An injection device (100) comprising:
a syringe (5) comprising a barrel (4) having a medicament fluid disposed therein, a hub (2) disposed at a distal end of the barrel (4), and a needle (6) coupled to a distal end of the hub (2); and
the needle cover (10) according to any of claims 1-10 disposed on a distal end of the syringe (5) such that at least a part of the inner surface (14) of the needle cover body (12) is in contact with and sealingly engaged with the needle (6) and an exterior surface (3) of the distal end of the syringe (5).

12. The injection device (100) of claim 11, wherein the barrel (4) is made of glass.

13. The injection device (100) of claim 11 or 12, wherein a pull out force for removing the needle cover (10) from the syringe (5) is in a range from 7 N to 16 N, preferably 7-10 or 10-15 N, when measured between 0 and 90 days after the needle cover is subjected to a sterilization process.

14. A method of forming the needle cover (10) according to any of claims 1-10, comprising injection molding the needle cover body (12) using a mold having a surface roughness in a range from 2 µm to 12 µm, inclusive.
